# EUROPÄISCHE PATENTSCHRIFT

(11) **EP 2 595 944 B1**
(45) Veröffentlichungstag und Bekanntmachung des Hinweises auf die Patenterteilung: **11.06.2014**
(21) Anmeldenummer: 11732461.6
(22) Anmeldetag: 15.07.2011
(51) Int. Cl.: C07C 2/78

(54) **VERFAHREN ZUR HERSTELLUNG VON ACETYLEN NACH DEM SACHSSE-BARTHOLOME-VERFAHREN**
METHOD FOR PRODUCING ACETYLENE ACCORDING TO THE SACHSSE-BARTHOLOME METHOD
PROCÉDÉ DE FABRICATION D'ACÉTYLÈNE PAR LE PROCÉDÉ SACHSSE-BARTHOLOMÉ

(30) Priorität: 20.07.2010 EP 10170151
(43) Veröffentlichungstag der Anmeldung: 29.05.2013
(73) Patentinhaber: BASF SE, 67056 Ludwigshafen (DE)
(72) Erfinder: KOENIGSMANN, Lucia, 70197 Stuttgart (DE); VICARI, Maximilian, 67117 Limburgerhof (DE); HEIDEMANN, Thomas, 68519 Viernheim (DE); GROSSSCHMIDT, Dirk, 68259 Mannheim (DE); MICHEL, Juergen, 67067 Ludwigshafen (DE)
(86) Internationale Anmeldenummer: PCT/EP2011/062125
(87) Internationale Veröffentlichungsnummer: WO 2012/010508

(56) Entgegenhaltungen:
- EP-A2- 1 041 037
- EP-A2- 1 462 160
- DE-A1-102008 061 611

## Beschreibung

Die Erfindung betrifft ein Verfahren zur Herstellung von Acetylen nach dem Sachsse-Bartholome-Verfahren.

Acetylen ist ein hochreaktives, farb- und geruchloses Gas. Viele Zwischenprodukte wie zum Beispiel Butandiol, Vinylether oder Vinylmonomere werden aus Acetylen hergestellt. Acetylen kann zum Beispiel nach dem Sachsse-Bartholome-Verfahren, durch Verbrennung eines Erdgas/Sauerstoff-Gemisches bei etwa 1700 °C unter Erhalt von Spaltgas, das als Nebenprodukte Synthesegas (H₂/CO), CO₂, Koks und Rohnaphthalin enthält, gewonnen werden. Das heiße Spaltgas wird nach der Verbrennung in mehreren Stufen in Brennerkolonnen abgekühlt. In einer ersten Stufe wird die heiße Reaktionsmischung mit einem Öl gequencht. In dieser ersten Quenchstufe wird das Spaltgas innerhalb weniger Millisekunden auf ca. 250 °C abgekühlt. Dabei bilden sich eine Vielzahl aromatischer und polycyclischer Verbindungen. Etwa ein Drittel dieser Verbindungen sind Monomere, hauptsächlich Styrol, Inden und Derivate hiervon. Diese hochreaktiven monomeren Verbindungen zeigen eine große Neigung Polymere zu bilden, was in bestimmten Anlagenteilen zu Problemen mit Polymerablagerungen führt.

Um diese Probleme zu reduzieren, wurden verschiedene Vorschläge zur Stabilisierung von Gemischen aus der Acetylen-Herstellung durch Zugabe von Polymerisationsinhibitoren gemacht. Geeignet sind hierfür insbesondere Polymerisationsinhibitoren ausgewählt aus der Gruppe bestehend aus N-Oxylen, aromatischen Aminen, aliphatischen Aminen, Phenolen und Mischungen hiervon, wie beispielsweise in DE 10 2008 061 611 vorgeschlagen.

Die Probleme in Verbindung mit Polymerablagerungen können jedoch auch vermindert oder vermieden werden, indem die polymerisationsfähigen Komponenten, insbesondere Styrol und Inden aus dem Produktgemisch, das durch Quenchen des Spaltgases mit Pyrolyseöl anfällt, entfernt werden, insbesondere durch Selektivhydrierung.

Aufgabe der Erfindung war es, ein technisch einfaches und wirtschaftliches Verfahren zur Selektivhydrierung von Komponenten aus mit Pyrolyseöl gequenchten Spaltgasen aus der Acetylen-Herstellung, die zu Problemen mit Polymerablagerungen führen würden, zur Verfügung zu stellen.

Die Lösung besteht in einem Verfahren zur Herstellung von Acetylen nach dem Sachsse-Bartholome-Verfahren durch Verbrennung eines Erdgas/Sauerstoff-Gemisches in einem oder mehreren Brennern unter Erhalt eines Spaltgases, das in zwei oder mehreren Stufen in Brennerkolonnen abgekühlt wird, wobei jedem Brenner jeweils eine oder mehrere Brennerkolonnen zugeordnet sind, und wobei das Spaltgas in der ersten Abkühlstufe mit Pyrolyseöl gequencht wird, unter Erhalt einer Styrol, Inden, Benzol, Toluol und Xylol enthaltenden Leichtsiederfraktion aus der einen oder den mehreren Brennerkolonnen, die mit Direktkühlwasser abgekühlt und in einem Phasenscheider in eine wässrige Phase und eine Styrol, Inden, Benzol, Toluol und Xylol enthaltende organische Phase aufgetrennt wird, die auf den Kopf der einen oder der mehreren Brennerkolonnen als Rücklauf aufgegeben wird, das dadurch gekennzeichnet ist, dass die organische, Styrol, Inden, Benzol, Toluol und Xylol enthaltende Phase aus dem Phasenscheider einer Selektivhydrierung an einem Katalysator zugeführt wird, der mindestens ein Platingruppenmetall auf einem anorganischen Metalloxid als Träger aufweist, enthaltend 0,05 bis 5 Gew.-% Platingruppenmetall, bezogen auf das Gesamtgewicht des Katalysators, und wobei die Selektivhydrierung bei einem Druck im Bereich von 1 bis 40 bar und einer Temperatur im Bereich von 25 bis 150 °C durchgeführt wird.

Nach dem Sachsse-Bartholome-Verfahren wird Acetylen durch Verbrennung eines Erdgas/Sauerstoff-Gemisches in einem oder mehreren Brennern erzeugt. Hierbei wird die für die endotherme Acetylenbildung erforderliche hohe Reaktionstemperatur durch partielle Oxidation des eingesetzten Erdgases mit reinem Sauerstoff bereitgestellt.

Dabei entsteht ein Spaltgas mit typischerweise folgender Zusammensetzung:

| | |
|---|---|
| H₂ | 56 Vol-% |
| CH₄ | 5 Vol-% |
| C₂H₂ | 7,5 Vol-% |
| CO | 26 Vol-% |
| CO₂ | 3,5 Vol-% |
| höhere Kohlenwasserstoffe | 1 Vol-%, |
| Rest Stickstoff, Sauerstoff und Argon, bis auf insgesamt | 100 Vol-%. |

Zur Gewährleistung einer hohen Selektivität ist es erforderlich Erdgas und Sauerstoff getrennt in mit Erdgas befeuerten Erhitzern auf 600 °C aufzuheizen. Nach der Mischeinrichtung und Passieren des Brennerblocks erfolgt die partielle Oxidation des Gasgemisches. So entsteht innerhalb weniger Tausendstelsekunden das acetylenhaltige Spaltgas in einer Flammenreaktion bei 1500 bis 1700°C.

Das hierbei erhaltene heiße Spaltgas wird in zwei oder mehreren Brennerkolonnen abgekühlt, wobei in einer ersten Abkühlstufe mit Pyrolyseöl möglichst schlagartig auf ca. 240 °C abgeschreckt (gequencht) wird. Pyrolyseöl ist ein Sumpfstrom aus Steamcrackern und besteht fast ausschließlich aus aromatischen Verbindungen.

Trotz der kurzen Verweilzeit zerfällt ein Teil des gebildeten Acetylens zu Wasserstoff und Ruß, der vom Quenchöl aufgenommen wird.

In den Brennerkolonnen wird das gequenchte Spaltgas weiter schrittweise bis auf ca. 70 °C abgekühlt und verlässt anschließend die Brennerkolonnen zur weiteren Aufarbeitung in die Gasfraktionen Roh-Acetylen (zur Acetylenreinigung, die in eine Butandiol-Anlage integriert sein kann), Armgas (CO/H₂-Synthesegas zur Methanol-Anlage) sowie höhere Acetylene, die unter Dampferzeugung verbrannt werden.

Durch die Berührung des Pyrolyseöls mit den heißen Spaltgasen findet eine Crackung des Pyrolyseöls statt, wobei Koks (Ruß) sowie eine Vielzahl aromatischer und polycyclischer Verbindungen entsteht, insbesondere Benzol, Toluol, Ethylbenzol, m/p/o-Xylole, Methylethylbenzol, Trimethylbenzole, Naphthalin und Mischungen davon als nicht polymerisierbare Komponenten und Ethinyl-Benzol, Styrol, 2,3,4-Methylstyrole, Inden, Methylindene, Dicyclopenten, Ethylen, Propylen, Acetylen, Propin, Propadien, Buten, Butadien und Mischungen als polymerisierbare Verbindungen.

Daher muss ständig frisches Pyrolyseöl vom Steamcracker zugefahren werden.

Um stets sicherzustellen, dass der Rußgehalt eine vorgegebene Maximalkonzentration nicht übersteigt, muss im Quenchkreis ständig ein Teilstrom des Pyrolyseöls in mit Erdgas befeuerten Regenerieröfen regeneriert, d.h. in Acetylenkoks und Pyrolyseöl aufgetrennt werden. Hierbei verkokt auch ein Teil des Pyrolyseöls. Der Acetylenkoks wird ausgeschleust und stellt ein Verkaufsprodukt dar.

Der letzte Abkühlschritt in den Brennerkolonnen erfolgt mit einem Benzol, Toluol und Xylol enthaltenden Gemisch, das als Leichtsiederfraktion beim Quenchen des Spaltgases mit Pyrolyseöl entsteht.

Anschließend wird das Spaltgas mit Direktkühlwasser auf ca. 40 °C abgekühlt, in einem Phasenscheider in eine wässrige Phase sowie eine organische Phase aufgetrennt, die neben Benzol, Toluol und Xylol auch hohe Anteil an Styrol und Inden enthält, mit der Folge, dass dies zur Polymerisation im oberen Bereich der Brennerkolonnen sowie im Direktkühlwasserkreislauf führen würde.

Daher werden vor der Rückführung dieses Stromes auf den Kopf der Brennerkolonnen die Komponenten, die zur Polymerisation führen würden, insbesondere Styrol und Inden, einer Selektivhydrierung zugeführt. Hierzu wird der gesamte, Benzol, Toluol und Xylol enthaltende Strom der Anlage durch die Selektivhydrierung gefahren. Der nun durch Selektivhydrierung stabilisierte Strom kann als Rücklauf auf den Kopf der Brennerkolonnen zurückgeführt oder in die Aromatengewinnung geleitet werden.

Die organische, Styrol, Inden, Benzol, Toluol und Xylol enthaltende Phase aus dem Phasenscheider weist bevorzugt die folgende Zusammensetzung auf:
8 bis 18 Gew.-% Styrol,
5 bis 10 Gew.-% Inden,
15 bis 20 Gew.-% der Summe aus ortho-, para- und meta-Xylol,
15 bis 20 Gew.-% Toluol und
8 bis 10 Gew.-% Benzol,
wobei die Summe der Komponenten 100 Gew.-% beträgt.

Eine typische Zusammensetzung für die organische Phase aus der der Quenchstufe mit Pyrolyseöl nachgeschalteten Phasentrennung ist:
8 bis 10 Gew.-% Benzol,
15 bis 20 Gew.-% Toluol,
1 bis 5 Gew.-% Ethylbenzol,
15 bis 20 Gew.-% m/p/o-Xylole,
5 bis 10 Gew.-% Methylethylbenzol,
1 bis 5 Gew.-%Trimethylbenzole,
0 bis 1 Gew.-% Butadien,
5 bis 10 Gew.-% Ethinyl-Benzol,
14 bis 18 Gew.-% Styrol,
0 bis 5 Gew.-% 2,3,4-Methylstyrol,
5 bis 10 Gew.-% Inden,
0 bis 4 Gew.-% Methylinden,
0 bis 4 Gew.-% Dicyclopenten und
1 bis 5 Gew.-% Naphthalin,
wobei die Summe der Komponenten 100 Gew.-% ergibt.

Die in dem erfindungsgemäßen Verfahren eingesetzten aromatischen Verbindungen stammen somit aus der Oxidation von Erdgas, die parallel bei der Herstellung von Acetylen aus Erdgas und Sauerstoff stattfindet, sowie aus dem Quenchprozess, bei dem wenigstens ein Teil des eingesetzten Quenchöls thermisch gecrackt wird.

Unter Selektivhydrierung der organischen, Styrol, Inden, Benzol, Toluol und Xylol enthaltenen Leichtsiederfraktion aus dem der Quenchstufe mit Pyrolyseöl nachgeschalteten Phasenscheider wird vorliegend die Hydrierung von Styrol, Inden und deren Derivaten unter Erhalt der aromatischen Kerne sowie die Hydrierung von Dienen zu den entsprechenden Monoenen verstanden.

Die Selektivhydrierung wird an einem geträgerten Katalysator durchgeführt, der ein anorganisches Metalloxid als Träger aufweist und als Aktivmasse mindestens ein Platingruppenmetall, wobei der Anteil des Platingruppenmetalls 0,05 bis 5 Gew.-%, bezogen auf die Gesamtmasse des Katalysators, beträgt. Die Selektivhydrierung wird bei einem Druck im Bereich von 10 bis 70 bar Überdruck, bevorzugt bei einem Druck im Bereich von 20 bis 40 bar Überdruck, besonders bevorzugt bei 30 bar Überdruck, betrieben. Die Temperatur der Selektivhydrierung liegt im Bereich von 25 bis 150 °C, bevorzugt 40 bis 120 °C, vorzugsweise 60 bis 100 °C, insbesondere 80 bis 100 °C.

Der eingesetzte Katalysator enthält mindestens ein Platingruppenmetall auf einem anorganischen Metalloxid als Träger, wobei der Gehalt an Platingruppenmetall 0,05 bis 5 Gew.-%, vorzugsweise 0,1 bis 2,5 Gew.-%, insbesondere 0,2 bis 1 Gew.-%, insbesondere 0,2 bis 0,4 Gew.-% beträgt. Besonders bevorzugt wird als Platingruppenmetall Palladium eingesetzt. Bis zu 20 Gew.-%, vorzugsweise bis zu 10 Gew.-% des Palladiums können dabei durch andere Platingruppenmetalle ersetzt sein. Besonders bevorzugt enthält der Katalysator nur Palladium als Aktivmetall. Der Träger kann aus beliebigen geeigneten anorganischen Metalloxiden ausgewählt sein. Bevorzugt wird als Katalysatorträger Aluminiumoxid, Titandioxid, Zirkonoxid, Siliciumdioxid oder ein Gemisch aus zweien oder mehreren davon eingesetzt. Besonders bevorzugt enthält der Träger Aluminiumoxid, insbesondere handelt es sich um einen Al₂O₃-Träger, beispielsweise einen γ-Aluminiumoxidträger.

Der Katalysatorträger weist eine Porosität von vorzugsweise 0,2 bis 1,0 ml/g, besonders bevorzugt 0,3 bis 0,6 ml/g auf. Der Median des Porenvolumens liegt vorzugsweise im Bereich von 5 bis 20 nm, vorzugsweise 7,5 bis 12,5 mm.

Da der Edelmetallkatalysator durch Kohlenmonoxid desaktiviert werden kann, wird die organische, Styrol, Inden, Benzol, Toluol und Xylol enthaltende Phase aus dem Phasenscheider bevorzugt vor der Zuführung zur Selektivhydrierung von darin enthaltenen Resten an Kohlenmonoxid bis auf 5 Gew.-%ppm, bezogen auf das Gesamtgewicht der organischen Phase aus dem Phasenscheider, besonders bevorzugt bis auf 1 Gew.-ppm, bezogen auf das Gesamtgewicht der organischen Phase aus dem Phasenscheider, abgereichert.

Die Abreicherung an Kohlenmonoxid erfolgt bevorzugt durch Strippen mit Wasserstoff.

Die Abreicherung an Kohlenmonoxid kann weiter bevorzugt durch Destillieren erfolgen.

Im erfindungsgemäßen Verfahren kann es über längere Zeiträume zu einer Deaktivierung des Katalysators kommen, beispielsweise, wenn größere Mengen an Styrol im Einsatzgemisch vorliegen. Eine Regenerierung des Katalysators kann nach bekannten Verfahren erfolgen. Beispielsweise kann der Katalysator mit Stickstoff gespült und anschließend unter Stickstoff auf Temperaturen im Bereich von 100 bis 300 °C, besonders bevorzugt 150 bis 250 °C erhitzt werden. Anschließend kann Stickstoff durch überhitzten Wasserdampf ersetzt werden, und es kann auf Temperaturen von 300 bis 400 °C weiter erhitzt werden. Beim Erreichen dieser Temperatur kann ein Teil des Wasserdampfs durch Luft ersetzt werden, beispielsweise 1 bis 10 Vol.-%. Danach kann das Katalysatorbett abgekühlt werden, wobei Wasserdampf wiederum durch Stickstoff ersetzt wird. Vor einem weiteren Einsatz des Katalysators sollte dieser nochmals reduziert werden. Die Regenerierung des Katalysators wird bevorzugt bei einem Druck im Bereich von 1 bis 5 bar, insbesondere 2 bis 4 bar durchgeführt.

Im erfindungsgemäßen Verfahren wurde eine besonders günstige Stelle in der Anlage zur Herstellung von Acetylen nach dem Sachsse-Bartholomé-Verfahren und Aufarbeitung des hierbei erhaltenen Gasgemisches für die Selektivhydrierung von darin enthaltenen hochreaktiven Monomeren, die zu Problemen mit Polymerablagerungen führen.

In der Anlage wird ein großer Rückführstrom, von typischerweise 40 m³/h, der überwiegend Benzol, Toluol und Xylol enthält, auf den Kopf der einen oder der mehreren Brennerkolonnen als Rücklauf aufgegeben. In diesen Strom mischt sich ein vergleichsweise kleiner Strom von typischerweise etwa 500 kg/h beim Quenchen des Spaltgases mit Pyrolyseöl entstehendes Benzol, Toluol und Xylol sowie hochreaktive Monomere, insbesondere Styrol und Inden enthaltendes Gemisch. Dies bedeutet, dass der Strom, der der Selektivhydrierung zugeführt wird, die beim Quenchen mit Pyrolyseöl anfallenden hochreaktiven polymerisierbaren Verbindungen in hoher Verdünnung enthält. Dadurch führt die stark exotherme Selektivhydrierung zu einer lediglich geringen Temperaturerhöhung von in der Regel maximal 5 K, und gestaltet sich dadurch relativ einfach, insbesondere weil keine besonderen Maßnahmen zur Auskopplung der Hydrierwärme erforderlich sind. Diese wären ansonsten notwendig, um zu verhindern, dass der Hydrierreaktor durchgeht. Das Verfahren ist daher sicherheitstechnisch und energetisch besonders vorteilhaft und schonend für den Katalysator.

Bevorzugt wird die Selektivhydrierung in einem Hydrierreaktor durchgeführt, der mit einer unter den Reaktionsbedingungen der Selektivhydrierung inerten Flüssigkeit, insbesondere Benzol, Toluol oder einem selektiv hydrierten Benzol, Toluol und XylolGemisch angefahren wird, und dem allmählich das der Selektivhydrierung zu unterziehende Styrol, Inden, Benzol, Toluol und Xylol enthaltende Gemisch, zugeführt wird. Durch diese Vorgehensweise wird die Gefahr einer Durchgehreaktion durch eine zu starke Temperaturerhöhung im Hydrierreaktor vermieden.

Bevorzugt wird das Verfahren in der Weise durchgeführt, dass die Zuführung von Wasserstoff in den Hydrierreaktor dergestalt erfolgt, dass ein zumindest 10%iger Wasserstoffüberschuss, bezogen auf die Summe aus dem chemischen Wasserstoffverbrauch und dem unter den Reaktionsbedingungen der Selektivhydrierung im Hydrierreaktor gelösten Wasserstoff sichergestellt wird. Insbesondere soll ein Wasserstoffpartialdruck von mindestens 5 bar, bevorzugt von mindestens 18 bar und von maximal 50 bar im Hydrierreaktor gewährleistet sein.

Durch den Wasserstoffüberschuss wird unter anderem die Durchströmung durch den Hydrierreaktor und die Austragung von Inerten gewährleistet, sowie sichergestellt, dass der Katalysator aktiv bleibt.

Die Erfindung wird im Folgenden anhand eines Ausführungsbeispiels sowie einer Figur näher erläutert.

### Ausführungsbeispiel

Eine Benzol, Toluol und Xylol enthaltende Leichtsiederfraktion aus einer Brennkolonne, das durch Abkühlen eines Spaltgases durch Quenchen mit Pyrolyseöl erhalten wurde, enthaltend 17 Flächen-% Styrol und 11 Flächen-% Inden von 3,5 g/h wurde einem Hydrierreaktor zugeführt, der bei einer Temperatur von 60°C betrieben wurde, war mit einem Katalysator H0-55, d.h. einem Schalenkatalysator, enthaltend 0,27 Gew.-% Palladium auf γ-Aluminiumoxid, eine BET-Oberfläche von 240 m²/g und einem Porenvolumen von 0,45 ml/g.

Der Hydrierreaktor war ein Rohrreaktor mit einem Innendurchmesser von 6 mm, und der im Eintrittsbereich in einer Aufheizzone mit Metallkugeln und anschließend mit dem oben beschriebenen Katalysator bestückt war.

Der Katalysator wurde im Hydrierreaktor vor dem Anfahren mit Wasserstoff bei erhöhter Temperatur aktiviert, und hierzu bei 120°C und 10 ml/h Wasserstoff für 12 h drucklos reduziert.

Die Reaktion wurde angefahren bei 60 °C / 20 bar für 53 h. Dabei wurde überraschenderweise festgestellt, dass der Katalysator rasch desaktiviert (Styrol im Produkt von 0 auf 0,25 FI.-%).

Nach 53 Betriebsstunden wurde die Temperatur auf 80 °C erhöht und dabei über 500 h ein > 98 % Umsatz von Styrol zu Ethylbenzol auf > 96 % Umsatz von Inden zu Indan erhalten.

Nach 500 h wurde der Druck auf 30 bar erhöht und die Reaktion weitere 1600 h gefahren, dabei blieb der Umsatz von Styrol zu Ethylbenzol > 98 % bzw. > 96 % von Inden zu Indan.

Nach insgesamt 2153 h wurde die Temperatur auf 100 °C erhöht. Dabei stieg der Umsatz von Styrol zu Ethylbenzol auf > 99 % bzw. > 98 % von Inden zu Indan. Nach 2700 h wurde die Reaktion gestoppt, ohne dass der Katalysator deutlich desaktivierte.

Die Flüssigkeitsproben wurden durch GC untersucht. Als Säule wurden 150 m Pertocol FD 1 µm, ID 0,25 mm, 35 °C/20 min, 2 °C/min bis 140 °C, 4 °C/Min bis 250 °C/40 min eingesetzt.

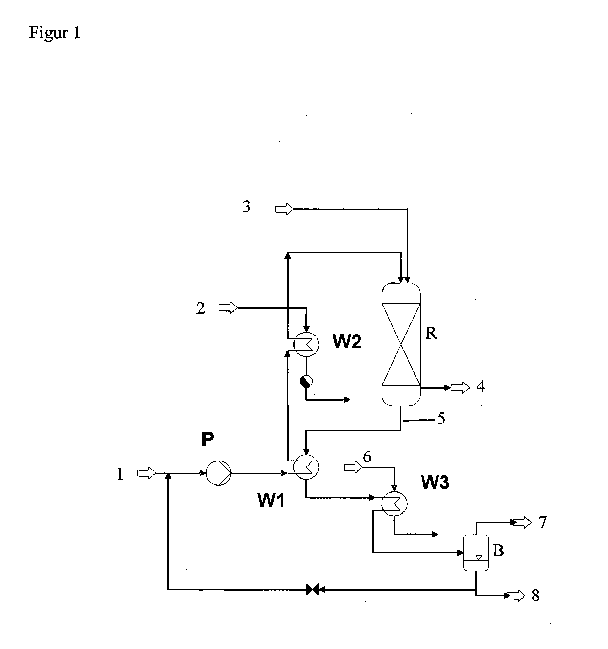

Die einzige Figur 1 zeigt die schematische Darstellung einer bevorzugten Anlage zur Selektivhydrierung nach dem erfindungsgemäßen Verfahren.

Nach einer Kohlenmonoxidabtrennung auf einen Kohlenmonoxid-Restgehalt von unter 5 Gew.-ppm wird der Styrol, Inden, Benzol, Toluol und Xylol enthaltende Einsatzstrom, Strom 1, mittels der Einsatzpumpe P auf den erforderlichen Reaktionsdruck von ca. 3 bar Überdruck gebracht. Dieser wird einem Gegenstromwärmetauscher W1 zugeführt, darin vorerwärmt und anschließend dem Spitzenaufheizer W2 zugeführt, worin die erforderliche Reaktoreintrittstemperatur von 80 °C, bei Start Of Run (SOR) bis 140 °C bei End Of Run (EOR) eingestellt wird. Dieser Einsatzstrom wird zusammen mit Wasserstoff, Strom 3, von oben nach unten über eine Katalysatorschüttung mengengeregelt auf den Hydrierreaktor R aufgegeben.

Es ist möglich, zwei parallel geschaltete Hydrierreaktoren einzusetzen, um eine in-situ-Regeneration vorzunehmen. So kann stets ein Reaktor in Betrieb sein, während der zweite Reaktor regeneriert wird.

Im Sumpf des Hydrierreaktors R trennt sich die Flüssigphase, das selektiv hydrierte, Benzol, Toluol und,Xylol enthaltende Gemisch von der überschüssigen Gasphase, die auch bei EOR-Bedingungen zu über 95 Vol.-% aus Wasserstoff besteht. Um den Katalysator aktiv zu halten, soll mindestens 10 % des chemischen Wasserstoffverbrauches als Abgas, Strom 4, aus dem Hydrierreaktor R druckgeregelt abgefahren werden. Damit wird auch der Druck im Reaktor eingestellt. Das Reaktorabgas, Strom 4, wird dem Spaltgassystem der Acetylenanlage zugeführt. Der Produktstrom 5, der aus dem Sumpf des Hydrierreaktors R abgezogen wird, wird nach Abkühlen im Gegenstromwärmetauscher W1 mit dem Einsatzstrom und weiterem Abkühlen auf ca. 40 °C im Produktkühler W3, der mit Kühlwasser, Strom 6, betrieben wird, in den Abscheider B auf ca. 4 bar Überdruck entspannt. Der Flüssiganfall, das selektiv hydrierte, Benzol, Toluol und Xylol enthaltende Produktgemisch, wird als Strom 8 abgezogen und auf die Brennerkolonnen der Anlage als Rücklauf verteilt. Der Überschuss kann einer Aromatengewinnung zugeführt werden.

Der Abscheider B dient auch dazu, die Selektivhydrierung mit dem bestehenden Hold-Up der Acetylenanlage mit hoch monomerhaltigem Benzol, Toluol und Xylol enthaltenden Gemisch anzufahren. Hierzu wird der Abscheider B beispielsweise mit Toluol oder Pyrolysebenzin-Vollraffinat gefüllt und über die Selektivhydrieranlage mittels der Einsatzpumpe P umgepumpt. Das hoch monomerhaltige Benzol, Toluol und Xylol enthaltende Gemisch aus den Trenngefäßen der Acetylenanlage wird sodann langsam zugegeben, so dass keine Temperaturerhöhung von mehr als 10 K im Reaktor auftritt. Selektiv hydriertes Benzol, Toluol und Xylol enthaltendes Gemisch wird zur Acetylenanlage ausgeschleust. Damit wird der gesamte, Benzol, Toluol und Xylol enthaltende Hold-Up der Acetylenanlage allmählich durch die Selektivhydrieranlage durchgesetzt.

## Patentansprüche

1. Verfahren zur Herstellung von Acetylen nach dem Sachsse-Bartholomé-Verfahren durch Verbrennung eines Erdgas/Sauerstoff-Gemisches in einem oder mehreren Brennern unter Erhalt eines Spaltgases, das in zwei oder mehreren Stufen in Brennerkolonnen abgekühlt wird, wobei jedem Brenner jeweils eine oder mehrere Brennerkolonnen zugeordnet sind, und wobei das Spaltgas in der ersten Abkühlstufe mit Pyrolyseöl gequencht wird, unter Erhalt einer Styrol, Inden, Benzol, Toluol und Xylol enthaltenden Leichtsiederfraktion aus der einen oder den mehreren Brennerkolonnen, die mit Direktkühlwasser abgekühlt und in einem Phasenscheider in eine wässrige Phase und eine Styrol, Inden, Benzol, Toluol und Xylol enthaltende organische Phase aufgetrennt wird, die auf den Kopf der einen oder der mehreren Brennerkolonnen als Rücklauf aufgegeben wird, **dadurch gekennzeichnet, dass** die organische, Styrol, Inden, Benzol, Toluol und Xylol enthaltende Phase aus dem Phasenscheider einer Selektivhydrierung an einem Katalysator zugeführt wird, der mindestens ein Platingruppenmetall auf einem anorganischen Metalloxid als Träger aufweist, enthaltend 0,05 bis 5 Gew.-% Platingruppenmetall, bezogen auf das Gesamtgewicht des Katalysators, und wobei die Selektivhydrierung bei einem Druck im Bereich von 1 bis 40 bar und einer Temperatur im Bereich von 25 bis 150 °C durchgeführt wird.

2. Verfahren nach Anspruch 1, **dadurch gekennzeichnet, dass** die organische, Styrol, Inden, Benzol, Toluol und Xylol enthaltende Phase aus dem Phasenscheider folgende Zusammensetzung aufweist:
8 bis 18 Gew.-% Styrol;
5 bis 10 Gew.-% Inden,
insgesamt 15 bis 20 Gew.-% aus der Summe von ortho-, para- und meta-Xylol,
15 bis 20 Gew.-% Toluol und
8 bis 10 Gew.-% Benzol,
wobei die Summe aller Komponenten 100 Gew.-% ergibt.

3. Verfahren nach Anspruch 1 oder 2, **dadurch gekennzeichnet, dass** der Katalysator Palladium auf Aluminiumoxid, Titandioxid, Zirkonoxid, Siliciumdioxid oder Gemischen davon als Träger enthält.

4. Verfahren nach einem der Ansprüche 1 bis 3, **dadurch gekennzeichnet, dass** die Selektivhydrierung in einem Festbett durchgeführt wird.

5. Verfahren nach einem der Ansprüche 1 bis 4, **dadurch gekennzeichnet, dass** die Selektivhydrierung in einem Hydrierreaktor durchgeführt wird, der mit einer inerten Flüssigkeit, insbesondere Benzol, Toluol oder einem selektiv hydrierten Benzol/Toluol/Xylol-Gemisch angefahren wird, und dem allmählich das der Selektivhydrierung zu unterziehende Gemisch, zugeführt wird.

6. Verfahren nach einem der Ansprüche 1 bis 5, **dadurch gekennzeichnet, dass** die organische, Styrol, Inden, Benzol, Toluol und Xylol enthaltende Phase aus dem Phasenscheider vor der Zuführung zur Selektivhydrierung von darin enthaltenen Resten an Kohlenmonoxid bis auf 5 Gew.-ppm, bezogen auf das Gesamtgewicht der organischen Phase aus dem Phasenscheider, bevorzugt bis auf 1 Gew.-ppm, bezogen auf das Gesamtgewicht der organischen Phase aus dem Phasenscheider, abgereichert wird.

7. Verfahren nach Anspruch 6, **dadurch gekennzeichnet, dass** die Abreicherung an Kohlenmonoxid durch Strippen mit Wasserstoff erfolgt.

8. Verfahren nach Anspruch 6, **dadurch gekennzeichnet, dass** die Abreicherung an Kohlenmonoxid durch Destillieren erfolgt.

9. Verfahren nach einem der Ansprüche 1 bis 8, **gekennzeichnet durch** eine Wärmeintegration in der Selektivhydrierung, indem die Wärme des Produktstromes aus der Selektivhydrierung zur Vorerwärmung des der Selektivhydrierung zu unterziehenden Styrol, Inden, Benzol, Toluol und Xylol enthaltenden Gemisches genutzt wird.

10. Verfahren nach einem der Ansprüche 1 bis 9, **dadurch gekennzeichnet, dass** die Zuführung von Wasserstoff in den Hydrierreaktor dergestalt erfolgt, dass ein zumindest 10 %iger Überschuss, bezogen auf die Summe aus dem chemischen Wasserstoffverbrauch und dem unter den Reaktionsbedingungen der Selektivhydrierung im Hydrierreaktor gelösten Wasserstoff, sichergestellt wird.

## Claims

1. A process for preparing acetylene by the Sachsse-Bartholom6 process by burning a natural gas/oxygen mixture in one or more burners to obtain a cracking gas which is cooled in two or more stages in burner columns, each burner having one or more burner columns assigned thereto, and said cracking gas being quenched with pyrolysis oil in the first cooling stage, to obtain a low boiler fraction comprising styrene, indene, benzene, toluene and xylene from the one or more burner columns, which is cooled with direct cooling water and separated in a phase separator into an aqueous phase and an organic phase which comprises styrene, indene, benzene, toluene and xylene and is introduced to the top of the one or more burner columns as a return stream, wherein the organic phase comprising styrene, indene, benzene, toluene and xylene from the phase separator is supplied to a selective hydrogenation over a catalyst which comprises at least one platinum group metal on an inorganic metal oxide as support, comprising 0.05 to 5% by weight of platinum group metal, based on the total weight of the catalyst, and wherein the selective hydrogenation is performed at a pressure in the range from 1 to 40 bar and a temperature in the range from 25 to 150°C.

2. The process according to claim 1, wherein the organic phase comprising styrene, indene, benzene, toluene and xylene from the phase separator has the following composition:
8 to 18% by weight of styrene;
5 to 10% by weight of indene,
a total of 15 to 20% by weight of the sum of ortho-, para- and meta-xylene,
15 to 20% by weight of toluene and
8 to 10% by weight of benzene,
where the sum of all components adds up to 100% by weight.

3. The process according to claim 1 or 2, wherein the catalyst comprises palladium on aluminum oxide, titanium dioxide, zirconium oxide, silicon dioxide or mixtures thereof as a support.

4. The process according to any of claims 1 to 3, wherein the selective hydrogenation is performed in a fixed bed.

5. The process according to any of claims 1 to 4, wherein the selective hydrogenation is performed in a hydrogenation reactor which is started up with an inert liquid, especially benzene, toluene or a selectively hydrogenated benzene/toluene/xylene mixture, and to which the mixture to be subjected to selective hydrogenation is gradually supplied.

6. The process according to any of claims 1 to 5, wherein the organic phase comprising styrene, indene, benzene, toluene and xylene from the phase separator, before being supplied to the selective hydrogenation, is depleted of residues of carbon monoxide present therein down to 5 ppm by weight, based on the total weight of the organic phase from the phase separator, preferably down to 1 ppm by weight, based on the total weight of the organic phase from the phase separator.

7. The process according to claim 6, wherein the depletion of carbon monoxide is effected by stripping with hydrogen.

8. The process according to claim 6, wherein the depletion of carbon monoxide is effected by distillation.

9. The process according to any of claims 1 to 8, **characterized by** thermal integration in the selective hydrogenation, by utilizing the heat of the product stream from the selective hydrogenation for preheating of the mixture which comprises styrene, indene, benzene, toluene and xylene and is to be subjected to the selective hydrogenation.

10. The process according to any of claims 1 to 9, wherein hydrogen is supplied to the hydrogenation reactor in such a way that an at least 10% excess, based on the sum of the chemical hydrogen consumption and the hydrogen dissolved in the hydrogenation reactor under the reaction conditions of the selective hydrogenation, is ensured.

## Revendications

1. Procédé de fabrication d'acétylène par le procédé Sachsse-Bartholomé par combustion d'un mélange gaz naturel/oxygène dans un ou plusieurs brûleurs pour obtenir un gaz craqué, qui est refroidi en deux étapes ou plus dans des colonnes de brûleur, une ou plusieurs colonnes de brûleur étant attribuées à chaque brûleur, et le gaz craqué étant trempé lors de la première étape de refroidissement avec une huile de pyrolyse, pour obtenir une fraction de composants de point d'ébullition faible contenant du styrène, de l'indène, du benzène, du toluène et du xylène à partir de la ou des colonnes de brûleur, qui est refroidie avec de l'eau de refroidissement direct et séparée dans un séparateur de phases en une phase aqueuse et une phase organique contenant du styrène, de l'indène, du benzène, du toluène et du xylène, qui est introduite en tant que reflux à la tête de la ou des colonnes de brûleur, **caractérisé en ce que** la phase organique contenant du styrène, de l'indène, du benzène, du toluène et du xylène issue du séparateur de phases est introduite dans une hydrogénation sélective sur un catalyseur, qui comprend au moins un métal du groupe du platine sur un oxyde métallique inorganique en tant que support, contenant 0,05 à 5 % en poids de métal du groupe du platine, par rapport au poids total du catalyseur, l'hydrogénation sélective étant réalisée à une pression dans la plage allant de 1 à 40 bar et à une température dans la plage allant de 25 à 150 °C.

2. Procédé selon la revendication 1, **caractérisé en ce que** la phase organique contenant du styrène, de l'indène, du benzène, du toluène et du xylène issue du séparateur de phases présente la composition suivante :
8 à 18 % en poids de styrène ;
5 à 10 % en poids d'indène ;
au total 15 à 20 % en poids de la somme d'ortho-, de para- et de méta-xylène ;
15 à 20 % en poids de toluène ; et
8 à 10 % en poids de benzène,
la somme de tous les composants étant de 100 % en poids.

3. Procédé selon la revendication 1 ou 2, **caractérisé en ce que** le catalyseur contient du palladium sur de l'oxyde d'aluminium, du dioxyde de titane, de l'oxyde de zirconium, du dioxyde de silicium ou leurs mélanges en tant que support.

4. Procédé selon l'une quelconque des revendications 1 à 3, **caractérisé en ce que** l'hydrogénation sélective est réalisée dans un lit fixe.

5. Procédé selon l'une quelconque des revendications 1 à 4, **caractérisé en ce que** l'hydrogénation sélective est réalisée dans un réacteur d'hydrogénation qui est démarré avec un liquide inerte, notamment du benzène, du toluène ou un mélange benzène/toluène/xylène hydrogéné sélectivement, et dans lequel le mélange à soumettre à l'hydrogénation sélective est introduit graduellement.

6. Procédé selon l'une quelconque des revendications 1 à 5, **caractérisé en ce que** la phase organique contenant du styrène, de l'indène, du benzène, du toluène et du xylène issue du séparateur de phases est appauvrie en les résidus de monoxyde de carbone qu'elle contient jusqu'à 5 ppm en poids, par rapport au poids total de la phase organique issue du séparateur de phases, de préférence jusqu'à 1 ppm en poids, par rapport au poids total de la phase organique issue du séparateur de phases, avant l'introduction dans l'hydrogénation sélective.

7. Procédé selon la revendication 6, **caractérisé en ce que** l'appauvrissement en monoxyde de carbone a lieu par extraction avec de l'hydrogène.

8. Procédé selon la revendication 6, **caractérisé en ce que** l'appauvrissement en monoxyde de carbone a lieu par distillation.

9. Procédé selon l'une quelconque des revendications 1 à 8, **caractérisé par** une intégration de chaleur dans l'hydrogénation sélective, la chaleur du courant de produits de l'hydrogénation sélective étant utilisé pour le préchauffage du mélange contenant du styrène, de l'indène, du benzène, du toluène et du xylène à soumettre à l'hydrogénation sélective.

10. Procédé selon l'une quelconque des revendications 1 à 9, **caractérisé en ce que** l'introduction d'hydrogène dans le réacteur d'hydrogénation a lieu de manière à assurer un excès d'au moins 10 %, par rapport à la somme de la consommation chimique d'hydrogène et de l'hydrogène dissous dans les conditions de réaction de l'hydrogénation sélective dans le réacteur d'hydrogénation.
